# EUROPÄISCHE PATENTSCHRIFT

(11) **EP 3 159 021 B1**
(45) Veröffentlichungstag und Bekanntmachung des Hinweises auf die Patenterteilung: **20.12.2017**
(21) Anmeldenummer: 16193431.0
(22) Anmeldetag: 12.10.2016
(51) Int. Cl.: A61M 1/00, A61B 10/02, A61M 5/31

(54) **VORRICHTUNG ZUR TRANSPLANTATION VON KÖRPERFETT**
DEVICE FOR TRANSPLANTATION OF ADIPOSE TISSUE
DISPOSITIF POUR LA TRANSPLANTATION DE TISSU ADIPEUX

(30) Priorität: 22.10.2015 DE 102015118011
(43) Veröffentlichungstag der Anmeldung: 26.04.2017
(73) Patentinhaber: Human Med AG, 19061 Schwerin (DE)
(72) Erfinder: Lindner, Bernd, 23617 Stockelsdorf (DE); Winkler, Konrad-Wenzel, 19417 Warin (DE); Wreth, Christian, 19067 Leezen (DE); Runow, Andreas, 19055 Schwerin (DE); Babinsky, Felix, 19057 Schwerin (DE); Schlee, Gernot, 19057 Schwerin (DE); Niklas, Frank, 19386 Lübz (DE)
(74) Vertreter: Gulde & Partner

(56) Entgegenhaltungen:
- US-A- 4 710 162
- US-A1- 2006 213 374
- US-A1- 2011 282 381

## Beschreibung

Die Erfindung betrifft eine Vorrichtung zur Transplantation von Körperfett und ein System mit der Vorrichtung.

Es ist dem Fachmann bekannt, dass Körperfett abgesaugt, entsorgt oder weiterverwendet werden kann. Hinsichtlich einer Weiterverwendung kommt häufig eine Transplantation von Körperfett in Betracht. Dem zuständigen Fachmann sind die hierfür erforderlichen Vorrichtungen und Verfahren grundsätzlich bekannt.

Häufig wird zur Trennung von Fett- und Bindegewebe zunächst ein Wasserstrahl mit entsprechendem Druck in den Körper eingeleitet. Es wird beispielsweise eine Tumeszenzlösung injiziert. Hierdurch können Fettzellen aus noch vorhandenem Bindegewebe ausgelöst werden. Das freigewordene Fettgewebe wird dann abgesaugt, aufbereitet (beispielsweise entwässert) und einem Patienten injiziert beziehungsweise reinjiziert. US 4 710 162 offenbart eine Vorrichtung zur Transplantation von Körperfett mit den Merkmalen des Oberbegriffs des Anspruchs 1.

Hinsichtlich bekannter Vorrichtungen und Verfahren stellt sich jedoch als nachteilig heraus, dass für die einzelnen Verfahrensschritte auch unterschiedliche Gerätschaften erforderlich sind. Es ist daher Aufgabe der vorliegenden Erfindung, eine Vorrichtung zur Transplantation von Körperfett anzugeben, die einfach, sicher und flexibel ist und mit der die Transplantation von Körperfett ganzheitlich realisierbar ist.

Diese Aufgabe wird durch die Gegenstände der unabhängigen Patentansprüche 1 und 9 gelöst.

Gegenstand der vorliegenden Erfindung ist eine Vorrichtung zur Transplantation von Körperfett, umfassend eine zumindest abschnittsweise hohlzylinderförmige Kammer, die durch einen an einer inneren Mantelfläche der Kammer führbaren Stempel in einen proximalen Abschnitt und einen von diesem strömungstechnisch entkoppelten distalen Abschnitt unterteilt wird, wobei sich an den Stempel ein Kolben anschließt, der ein distales Ende umfasst, welches eine durch den Stempel verlaufende strömungstechnische Verbindung zu dem proximalen Abschnitt der Kammer aufweist, und der Stempel im Bereich der strömungstechnischen Verbindung zumindest abschnittsweise als Filter ausgebildet ist und das distale Ende des Kolbens einen ersten schließbaren Medienanschluss umfasst und weiterhin an dem proximalen Abschnitt der Kammer Mittel zur Führung von Flüssigkeiten und/oder Fettgewebe angeschlossen oder anschließbar sind, wobei durch Versorgung des ersten Medienanschlusses in geöffnetem Zustand mit einem Unterdruck Flüssigkeit und/oder Fettgewebe in den proximalen Abschnitt der Kammer einsaugbar sind und/oder Flüssigkeit durch den Stempel hindurch einsaugbar ist. Erfindungsgemäß ist vorgesehen, dass der distale Abschnitt der Kammer einen zweiten Medienanschluss umfasst, so dass durch Versorgung des zweiten Medienanschlusses mit einem Überdruck, während der erste Medienanschluss geschlossen ist, Flüssigkeit und/oder Fettgewebe über eine Verlagerung des Stempels durch den proximalen Abschnitt der Kammer auspumpbar sind und/oder dass durch Versorgung des zweiten Medienanschlusses mit Überdruck, während der erste Medienanschluss zumindest geöffnet ist, Flüssigkeit und/oder Fettgewebe über die Verlagerung des Stempels durch den proximalen Abschnitt der Kammer auspumpbar sind und simultan Flüssigkeit durch den Stempel hindurch abführbar ist.

Dies bietet den Vorteil, dass die erfindungsgemäße Vorrichtung besonders einfach und sicher ist und derart flexibel gestaltet ist, dass alle Verfahrensschritte zur Transplantation von Körperfett mit ihr durchführbar sind.

Zur Verdeutlichung dieser Vorteile wird im Folgenden gemäß eines weiteren Aspekts der vorliegenden Erfindung ein Verfahren zur Transplantation von Körperfett beschrieben.

Die Bedeutung der Begriffe proximal und distal sind dem Fachmann grundsätzlich bekannt. Dennoch folgt hier aus Klarheitsgründen eine explizite Definition: Der Begriff proximal wird im Zusammenhang mit der vorliegenden Erfindung definiert als zur Körpermitte eines Patienten hin lokalisiert. Der Begriff distal wird im Zusammenhang mit der vorliegenden Erfindung definiert als von der Körpermitte des Patienten weg lokalisiert. Bei Vorrichtungsmerkmalen ist somit darauf abzustellen, ob ein bestimmtes Merkmal gegenüber einem anderen Merkmal für eine Verwendung dichter an der Körpermitte des Patienten oder weiter von dieser entfernt vorgesehen ist.

In einem ersten Verfahrensschritt wird dabei zunächst eine Infiltrationsflüssigkeit derart bereitgestellt, dass sie unter Druck in einen Bereich eines Entnahmeortes des Körperfetts einbringbar ist. Die erfindungsgemäße Vorrichtung zur Transplantation von Körperfett ist hierzu besonders vorteilhaft einsetzbar. So ist Infiltrationsflüssigkeit besonders einfach und sicher in den proxymalen Abschnitt der hohlzylinderförmigen Kammer einsaugbar. Zu diesem Zweck kann die Vorrichtung zur Transplantation von Körperfett beispielsweise mit dem proximalen Abschnitt in ein Reservoir von Infiltrationsflüssigkeit geführt werden und dann über den geöffneten ersten Medienanschluss mit einem Unterdruck versorgt werden. Somit ist eine einfache und sichere Bedienbarkeit der Vorrichtung gegeben. Die Vorrichtung ist auch besonders gut handhabbar, wodurch die Sicherheit in einem medizinischen Umfeld erhöht wird. Die Vorrichtung kann, auch ohne dass Mittel zur Führung von Flüssigkeiten und/oder Fettgewebe an dem proximalen Abschnitt angeschlossen sind, in das Reservoir von Infiltrationsflüssigkeit gehalten werden und diese einsaugen. Somit kann vorteilhaft ein großer Strömungsquerschnitt realisiert werden und es ist eine schnelle Befüllung möglich. Mit Vorteil ist eine Position des Kolbens der Vorrichtung an einen distalen Totpunkt oder an eine andere Position innerhalb der hohlzylinderförmigen Kammer einstellbar. Somit ist die einzusaugende Menge an Infiltrationsflüssigkeit vorteilhaft gut dosierbar. Ein weiterer Vorteil besteht darin, dass überschüssige Infiltrationsflüssigkeit durch den Filter des Stempels und den ersten Medienanschluss einfach abführbar ist. Ein weiterer Vorteil besteht darin, dass der erste Medienanschluss öffenbar und schließbar ist. Somit ist die Vorrichtung besonders sicher zwischen einem Zustand mit und ohne Möglichkeit zur äußeren Druckversorgung über den ersten Medienanschluss umschaltbar.

In einem zweiten Verfahrensschritt wird die Infiltrationsflüssigkeit mit einem Überdruck von vorzugsweise 0,5 bar bis 30 bar, weiterhin bevorzugt 1 bar bis 5 bar und besonders bevorzugt 1 bar bis 2 bar in den Bereich der Entnahmestelle des Körperfettes eingestrahlt, um dieses zu mobilisieren. Durch die Infiltrationsflüssigkeit wird das Fettgewebe gelockert und ist leichter aus dem Bindegewebe auszulösen. Letzteres kann beispielsweise durch Absaugen erfolgen. Die erfindungsgemäße Vorrichtung ist vorteilhafterweise mit unterschiedlichen Mitteln zur Führung von Flüssigkeiten und/oder Fettgewebe, die an dem proximalen Abschnitt der Kammer anschließbar sind, an verschiedene Infiltrationsaufgaben anpassbar und dadurch besonders flexibel. Beispielsweise können die Mittel eine Infiltrationskanüle mit beispielsweise 300 µm Düsendurchmesser umfassen, so dass die Infiltrationsflüssigkeit besonders einfach und sicher einstrahlbar ist. Zur Beaufschlagung der Infiltrationsflüssigkeit mit einem definierten Überdruck ist der erste Medienanschluss der Vorrichtung vorteilhaft einfach schließbar und der zweite Medienanschluss mit einem Überdruck versorgbar. Da der proxymale Abschnitt und der distale Abschnitt strömungstechnisch entkoppelt sind, wird besonders sicher eine Verlagerung des Stempels bewirkt, ohne dass Druckmedium in den proximalen Abschnitt gelangen kann. Während der Infiltration ist die Vorrichtung zudem besonders einfach und sicher handhabbar, was die Sicherheit erhöht.

In einem dritten Verfahrensschritt erfolgt ein Absaugen des ausgelösten Fettgewebes. Das Absaugen erfolgt vorzugsweise mit einem Unterdruck von vorzugsweise -900 mbar bis -300 mbar, besonders bevorzugt von -600 mbar bis -400 mbar und ganz besonders bevorzugt von -500 mbar. Mit Vorteil kann die Vorrichtung flexibel mit entsprechenden Mitteln zur Führung von Flüssigkeiten und/oder Fettgewebe ausgerüstet werden. Beispielsweise kann eine entsprechende Absaugkanüle mit beispielsweise 2 mm Düsendurchmesser an dem proximalen Abschnitt der Kammer angebracht werden. Der Vorgang des Absaugens ist mit der erfindungsgemäßen Vorrichtung besonders einfach und sicher durchführbar. Der erste Medienanschluss ist hierzu vorteilhaft einfach öffenbar und mit einem Unterdruck beaufschlagbar. Die erfindungsgemäße Vorrichtung bietet weiterhin den Vorteil, dass während des Absaugvorganges von Fettgewebe und Flüssigkeit mittels des Stempels, der abschnittsweise als Filter ausgebildet ist, Flüssigkeit durch den Stempel hindurch einsaugbar ist, wobei Fettgewebe in dem proximalen Abschnitt verbleibt, so dass allein die Flüssigkeit durch den ersten Medienanschluss abführbar ist. Mit der erfindungsgemäßen Vorrichtung lassen sich so ein Absaugen und Aufbereiten des Fettgewebes oder eines Stoffgemisches, umfassend wenigstens das Fettgewebe und die Flüssigkeit, in einem einzigen Verfahrensschritt durchführen.

In einem vierten Verfahrensschritt erfolgt eine Reinjektion des Körperfetts. Die Reinjektion erfolgt mit einem Überdruck von vorzugsweise 0,5 bar bis 30 bar, weiterhin bevorzugt 10 bar bis 15 bar, besonders bevorzugt 6 bar bis 9 bar und ganz besonders bevorzugt 7 bar. Die erfindungsgemäße Vorrichtung bietet den Vorteil, dass eine Reinjektion besonders einfach und sicher erfolgen kann. So lässt sich Körperfett besonders einfach und sicher aus dem proximalen Abschnitt der Kammer mit definiertem Druck ausbringen, in dem der zweite Medienanschluss mit einem Überdruck versorgt wird. Die Vorrichtung lässt sich besonders einfach und flexibel an unterschiedliche Reinjektionsaufgaben anpassen. So können die Mittel zur Führung von Flüssigkeiten und/oder Fettgewebe beispielsweise eine Reinjektionskanüle mit einem Düsendurchmesser von beispielsweise 1,4 mm umfassen. Besonders vorteilhaft ist es mit der erfindungsgemäßen Vorrichtung möglich, das Körperfett unter gleichzeitiger Trennung von Flüssigkeit und Fettgewebe aus dem proximalen Abschnitt der Kammer auszupumpen. Hierzu muss der erste Medienanschluss lediglich geöffnet sein. Da sich in der proximalen Kammer infolge einer Verlagerung des Stempels ein Druck aufbaut, wird das in dem proximalen Abschnitt befindliche Stoffgemisch, welches einen Restgehalt an Flüssigkeit und das Fettgewebe enthalten kann, gegen den Stempel gedrückt, der abschnittsweise als Filter ausgebildet ist. Somit ist noch vorhandene Flüssigkeit besonders einfach und sicher von dem Fettgewebe trennbar und durch den Filter abführbar, während allein das Fettgewebe aus dem proximalen Abschnitt in beispielsweise einen Bereich einer Implantationsstelle auspumpbar ist. Somit können mit der erfindungsgemäßen Vorrichtung vorteilhaft auch die Vorgänge des Reinjizierens und des Aufbereitens kombiniert werden. Vorteilhaft ist es auch möglich, den ersten Medienanschluss zusätzlich mit einem Unterdruck zu beaufschlagen. Somit ist eine Druckdifferenz in der strömungstechnischen Verbindung zwischen dem proximalen Abschnitt der Kammer und dem distalen Ende des Kolbens, also einer Druckdifferenz über den Stempel und damit dessen Filterwirkung zu erhöhen. Der Entwässerungseffekt für das Fettgewebe kann so vorteilhaft noch verbessert werden. Auch dies trägt vorteilhaft dazu bei, dass eine Transplantation von Körperfett mit der erfindungsgemäßen Vorrichtung besonders schnell und gleichzeitig sicher sowie einfach durchgeführt werden kann.

Im Folgenden werden bevorzugte Ausgestaltungen der erfindungsgemäßen Vorrichtung beschrieben. Dabei wird zur Verdeutlichung der Vorteile der Vorrichtung gelegentlich Bezug zu dem oben beschriebenen erfindungsgemäßen Verfahren genommen. Gegebenenfalls werden auch bevorzugte Ausgestaltungen des erfindungsgemäßen Verfahrens beschrieben.

In bevorzugter Ausgestaltung der erfindungsgemäßen Vorrichtung ist nun vorgesehen, dass der Stempel wenigstens eine weitere strömungstechnische Verbindung zwischen einer äußeren Mantelfläche des Stempels und einer Austrittsöffnung am distalen Ende des Kolbens aufweist.

Dies bietet den Vorteil, dass ein Überdruck, der infolge von Fertigungstoleranzen oder sonstigen Fehlereinflüssen aus dem distalen Abschnitt der Kammer zwischen die innere Mantelfläche der Kammer und den Stempel gelangt, durch diese weitere strömungstechnische Verbindung in die Umwelt abführbar ist. Die Sicherheit der Vorrichtung wird dadurch vorteilhaft erhöht, da eine Vermischung von Druckmedium mit Fettgewebe in dem proximalen Abschnitt stets vermieden wird.

In weiterer bevorzugter Ausgestaltung der vorliegenden Erfindung ist vorgesehen, dass als Mittel zur Führung von Flüssigkeiten und/oder Fettgewebe wenigstens eine Vorkammer mit wenigstens einer Bypassöffnung und/oder wenigstens eine Kanüle oder Kanüle mit Bypassöffnung an den proximalen Abschnitt der Kammer angeschlossen oder anschließbar sind.

Dies bietet den Vorteil, dass über die Bypassöffnung ein Absaugen von Fettgewebe aus dem Bereich der Entnahmestelle flexibel unterbrochen und fortgesetzt werden kann. Somit wird die Flexibilität und auch die Sicherheit der Vorrichtung vorteilhaft erhöht. Die Möglichkeit, unterschiedliche Kanülen an der Vorrichtung zu montieren, macht diese flexibel anpassbar an unterschiedlichste Anforderungen aus den oben genannten Verfahrensschritten Flüssigkeitsaufnahme, Infiltration, Absaugen von Fettgewebe und Injektion von Fettgewebe. Hierfür vorzusehene Kanülen sind dem Fachmann grundsätzlich bekannt.

In weiterer bevorzugter Ausgestaltung der vorliegenden Erfindung ist vorgesehen, dass die Vorkammer wenigstens einen Vorfilter zur Ausfilterung von Bindegewebsrückständen umfasst.

Dies bietet den Vorteil, dass eine Agglomeration von Bindegewebsrückständen und damit verbunden die Gefahr einer Verstopfung der Vorrichtung vermieden wird. Zuverlässigkeit und Sicherheit der Vorrichtung werden damit vorteilhaft erhöht.

In weiterer bevorzugter Ausgestaltung der vorliegenden Erfindung ist vorgesehen, dass der zweite Medienanschluss ein Ventil umfasst, welches sich in einem Sperrzustand befindet, wenn der Kolben sich an einem distalen Totpunkt befindet, und sich in einem Durchlasszustand befindet, wenn der Kolben sich nicht an einem distalen Totpunkt befindet.

Dies bietet den Vorteil, dass durch den Sperrzustand des Ventils ein über den ersten Medienanschluss in dem proximalen Abschnitt der Kammer erzeugtes Vakuum nicht gestört wird, selbst dann, wenn unerwünscht Undichtigkeiten im Bereich des Stempels und der inneren Mantelfläche der Kammer auftreten. Mit anderen Worten ausgedrückt, wenn unerwünscht, beispielsweise durch Fehlereinflüsse, eine fluidtechnische Verbindung zwischen dem proximalen Abschnitt der Kammer und dem distalen Abschnitt der Kammer zwischen dem Stempel und der inneren Mantelfläche der Kammer hindurch entsteht, kann selbst dann aufgrund des Sperrzustand des Ventils das Vakuum nicht durch ein durch den zweiten Medienanschluss einströmendes Medium zerstört werden. Weiterhin vorteilhaft ist durch den Durchlasszustand des Ventils sichergestellt, dass in den distalen Abschnitt der Kammer eingebrachter Überdruck über den zweiten Medienanschluss sicher wieder abführbar ist.

In weiterer bevorzugter Ausgestaltung der vorliegenden Erfindung ist vorgesehen, dass der Kolben an einem distalen Totpunkt durch Drehung um seine Längsachse verrastbar ist.

Dies bietet den Vorteil, dass eine sichere Arretierung des Kolben möglich ist. Dies ist beispielsweise dann besonders vorteilhaft, wenn der erste Medienanschluss mit einem Unterdruck beaufschlagt wird oder in dem proximalen Abschnitt der Kammer befindliche Flüssigkeit und/oder Fettgewebe in der Kammer gehalten werden soll.

Eine besonders bevorzugte Ausführungsform sieht vor, dass das Ventil des zweiten Medienanschlusses durch Drehung des Kolbens um seine Längsachse am distalen Totpunkt in seinen Sperrzustand schaltbar ist und gleichzeitig der Kolben arettierbar ist. Die Sicherheit der Vorrichtung wird dadurch signifikant erhöht.

In weiterer bevorzugter Ausgestaltung der vorliegenden Erfindung ist vorgesehen, dass eine Durchlassgrenze wenigstens eines als Filter ausgebildeten Abschnitts des Stempels zwischen 100 µm und 300 µm, besonders bevorzugt zwischen 100 µm und 250 µm und ganz besonders bevorzugt zwischen 100 µm und 190 µm liegt und/oder eine Durchlassgrenze wenigstens eines Vorfilters zwischen 0,5 mm und 3 mm, besonders bevorzugt zwischen 1 mm und 2 mm und ganz besonders bevorzugt bei 1,5 mm liegt.

Dies bietet den Vorteil, dass sowohl eine sichere Vorfilterung von größeren Bindegewebsrückständen als auch eine sichere Entwässerung des Fettgewebes möglich ist. Untersuchungen haben gezeigt, dass diese Durchlassgrenzen gleichzeitig vorteilhaft einen ausreichend großen Volumenstrom in und gegebenenfalls durch die Vorrichtung ermöglichen.

In weiterer bevorzugter Ausgestaltung der vorliegenden Erfindung ist vorgesehen, dass der zweite Medienanschluss mit einem Überdruck zwischen 0,5 bar und 30 bar, bevorzugt zwischen 1 bar und 15 bar, weiterhin bevorzugt 1 bar bis 5 bar und besonders bevorzugt 1 bar bis 2 bar beaufschlagbar ist und/oder der erste Medienanschluss mit einem Unterdruck zwischen -900 mbar und -300 mbar, bevorzugt von -500 mbar beaufschlagbar ist.

Dies bietet den Vorteil, dass bei der Transplantation von Körperfett die Verfahrensschritte der Infiltration und der Absaugung besonders gut und sicher mit der Vorrichtung durchführbar sind.

In weiterer bevorzugter Ausgestaltung der vorliegenden Erfindung ist vorgesehen, dass der zweite Medienanschluss mit einem Überdruck zwischen 0,5 bar und 30 bar, bevorzugt zwischen 10 bar und 15 bar und besonders bevorzugt mit 7 bar beaufschlagbar ist.

Dies bietet den Vorteil, dass bei der Transplantation von Körperfett der Verfahrensschritt der Reinjektion besonders gut und sicher mit der Vorrichtung durchführbar ist.

Ein weiterer Aspekt der vorliegenden Erfindung betrifft ein System, umfassend wenigstens eine erfindungsgemäße Vorrichtung zur Transplantation von Körperfett und wenigstens eine Medienversorgungseinrichtung.

Dies bietet den Vorteil, dass eine Versorgung der Vorrichtung mit Unterdruck, Überdruck oder Umgebungsdruck besonders einfach und sicher erfolgen kann. Insbesondere vorteilhaft lässt sich eine Versorgung mit verschiedenen Medien flexibel auf die Anforderungen anpassen. Rein beispielhaft kommen als Medium zur Versorgung mit einem Überdruck Kohlenstoffdioxid oder gereinigte Pressluft in Betracht. Zur Versorgung mit einem Unterdruck kann rein beispielhaft eine Vakuumpumpe verwendet werden. Zur Versorgung mit Umgebungsdruck kann die Medienversorgungseinrichtung beispielsweise Ventile zur Herstellung einer Verbindung mit der Umgebung aufweisen. Besonders vorteilhaft ist das System auch skalierbar, da beispielsweise mehrere Vorrichtungen zur Transplantation von Körperfett vorgesehen sein können.

In bevorzugter Ausgestaltung der vorliegenden Erfindung ist vorgesehen, dass das System weiterhin Mittel zur Trennung von gasförmigen Medienströmen und flüssigen Stoffströmen umfasst, die in einer Wirkverbindung zwischen der Vorrichtung zur Transplantation von Körperfett und der Medienversorgungseinrichtung angeordnet sind.

Dies bietet den Vorteil, dass das System selbst dann besonders sicher und zuverlässig ist, wenn beispielsweise Flüssigkeit durch den Stempel der Vorrichtung und den ersten Medienanschluss hindurch abgesaugt wird.

Weitere bevorzugte Ausgestaltungen der Erfindung ergeben sich aus den übrigen, in den Unteransprüchen genannten Merkmalen.

Die verschiedenen, in dieser Anmeldung genannten Ausführungsformen der Erfindung sind, sofern im Einzelfall nicht anders ausgeführt, mit Vorteil miteinander kombinierbar.

Die Erfindung wird nachfolgend in Ausführungsbeispielen anhand der zugehörigen Zeichnungen erläutert. Es zeigen:
- Figur 1: eine schematische Darstellung einer erfindungsgemäßen Vorrichtung zur Transplantation von Körperfett in einer bevorzugten Ausführungsform und
- Figur 2: eine schematische Darstellung eines erfindungsgemäßen Systems in einer bevorzugten Ausführungsform.

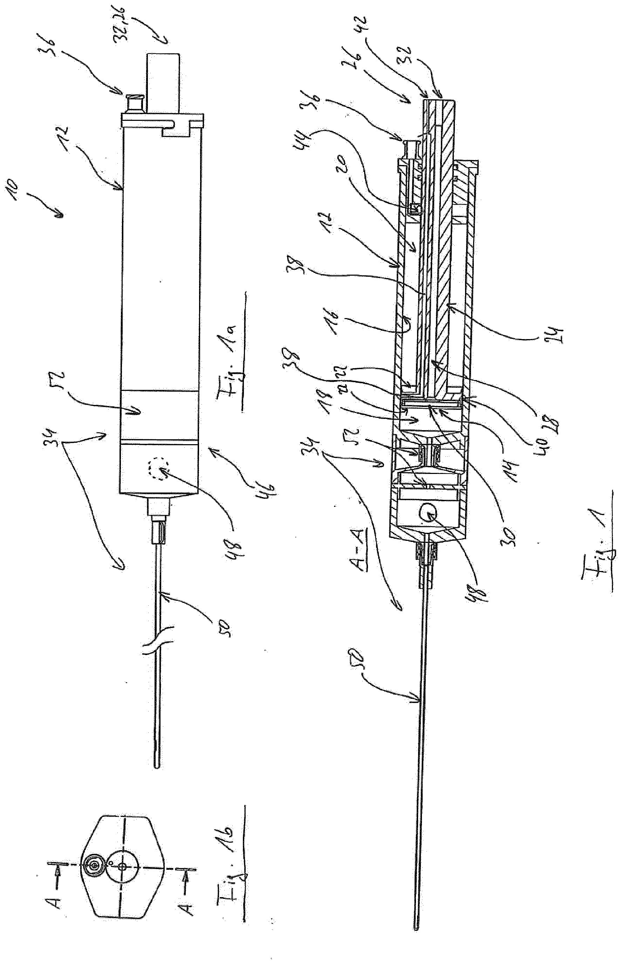

Figur 1 zeigt eine schematische Darstellung einer erfindungsgemäßen Vorrichtung zur Transplantation von Körperfett in einer bevorzugten Ausführungsform. Figur 1A zeigt die erfindungsgemäße Vorrichtung zum Transfer von Körperfett 10 in einer seitlichen Ansicht, Figur 1B eine Draufsicht. Erkennbar ist eine im Wesentlichen hohlzylinderförmige Kammer 12. Wie aus der Schnittansicht hervorgeht, ist die hohlzylinderförmige Kammer 12 durch einen Stempel 14, der an einer inneren Mantelfläche 16 der hohlzylinderförmigen Kammer 12 führbar ist, in einen proximalen Abschnitt 18 und einen von diesen strömungstechnisch entkoppelt distalen Abschnitt 20 unterteilt. Die strömungstechnische Entkopplung ist durch den Stempel 14, der entsprechende Dichtlippen 22 umfasst, realisiert. Eine dem proximalen Abschnitt 18 der Kammer 12 zugewandte Dichtlippe ist in diesem Ausführungsbeispiel ausgebildet, ein Eindringen von Flüssigkeit aus dem proximalen Abschnitt 18 in einen Bereich zwischen dem Stempel 14 und der inneren Mantelfläche 16 zu verhindern. Eine dem distalen Abschnitt 20 der Kammer 12 zugewandte Dichtlippe ist in diesem Ausführungsbeispiel ausgebildet, ein Eindringen von Pressluft oder Kohlenstoffdioxid aus dem distalen Abschnitt 20 in den Bereich zwischen dem Stempel 14 und der inneren Mantelfläche 16 zu verhindern. An den Stempel 14 schließt sich ein Kolben 24 an. Der Kolben 24 umfasst ein distales Ende 26, welches eine durch den Stempel 14 verlaufende strömungstechnische Verbindung 28 zu dem proximalen Abschnitt 18 aufweist. Der Stempel 14 ist in einem Bereich der strömungstechnischen Verbindung 28, welcher der proximalen Kammer 18 zugewandt ist, abschnittsweise als Filter 30 ausgebildet. Der Filter 30 weist eine Durchlassgrenze von 200 µm auf. Das distale Ende 26 des Kolbens 24 umfasst einen ersten schließbaren Medienanschluss 32. Dieser ist ausgelegt, mit einem Unterdruck zwischen -900 mbar und -300 mbar versorgt zu werden. Auch eine Versorgung mit Umgebungsdruck ist möglich. Der schließbare Medienanschluss 32 kann beispielsweise ein Absperrventil sein, welches hier nicht dargestellt ist. An dem proximalen Abschnitt 18 der Kammer 12 sind weiterhin Mittel 34 zur Führung von Flüssigkeiten und/oder Fettgewebe wechselbar angeschlossen. Durch Versorgung des ersten Medienanschlusses 32 in einem geöffneten Zustand mit einem Unterdruck ist Flüssigkeit und/oder Fettgewebe in den proximalen Abschnitt 18 der Kammer 12 einsaugbar. Ferner ist auch Flüssigkeit durch den Stempel 14, wo dieser als Filter 30 ausgebildet ist, hindurch einsaugbar. Weiterhin umfasst der distale Abschnitt 20 der Kammer 12 einen zweiten Medienanschluss 36. Dieser ist ausgebildet, mit einem Überdruck zwischen 0,5 bar und 30 bar versorgt zu werden. Auch eine Versorgung mit Umgebungsdruck ist möglich. Durch Versorgung des zweiten Medienanschlusses 36 mit einem Überdruck, während der erste Medienanschluss 32 über das beispielsweise Absperrventil geschlossen ist, ist in dem distalen Abschnitt 20 ein Druck aufbaubar. Infolge des Drucks ist der Stempel 14 verlagerbar, wodurch ein Volumen des proximalen Abschnitts 18 reduziert wird. Infolgedessen ist Flüssigkeit und/oder Fettgewebe aus dem proximalen Abschnitt 18 der Kammer 12 auspumpbar. Der Stempel 14 weist eine weitere strömungstechnische Verbindung 38 zwischen einer äußeren Mantelfläche 40 des Stempels 14 und einer Austrittsöffnung 42 am distalen Ende 20 des Kolben 24 auf. Durch die weitere strömungstechnische Verbindung 38 ist sichergestellt, dass ein in dem distalen Abschnitt 20 der Kammer 12 herrschender Überdruck selbst dann nicht in den proximalen Abschnitt 18 der Kammer 12 entweichen kann, wenn beispielsweise infolge von Fertigungstoleranzen oder anderen Fehlereinflüssen der Stempel 14 über seine äußere Mantelfläche 40 und die Dichtlippen 22 nicht vollständig druckdicht an der inneren Mantelfläche 16 der Kammer 12 führbar ist. Infolge solcher Fehlereinflüsse zwischen die Mantelflächen 16, 40 gelangendes Medium wird durch diese weitere strömungstechnische Verbindung 38 stets in die Umwelt abgeführt. Wenn gleichzeitig zur Versorgung des zweiten Medienanschlusses 36 mit dem Überdruck der erste Medienanschluss 32 zumindest geöffnet ist, so ist außerdem Flüssigkeit durch den Stempel 14 abführbar, während Fettgewebe und gegebenenfalls ein Restgehalt an Flüssigkeit im Wesentlichen gegenläufig zu der durch den Stempel 14 abführbaren Flüssigkeit aus dem proximalen Abschnitt 18 der Kammer 12 in Richtung der Mittel 34 beziehungsweise durch diese hindurch abführbar sind. Der zweite Medienanschluss 36 umfasst weiterhin ein Ventil 44, welches sich in einem Sperrzustand befindet, wenn der Kolben 24 sich an einem distalen Totpunkt befindet und der Kolben 24 dort durch Drehung um seine Längsachse verrastet ist. Das Ventil 44 kann rein exemplarisch als Kugelventil ausgeführt sein, bei welchem in einer Sperrstellung die Kugel durch eine Feder in eine Vertiefung des Kolben 24 gedrückt wird und so das Ventil 44 schließbar ist. Durch Drehung des Kolbens 24 werden die Kugel und die Vertiefung des Kolbens 24 zueinander ausgerichtet beziehungsweise durch entgegengesetzte Drehung voneinander gelöst. Das Ventil 44 sperrt oder ermöglicht ein Einströmen von Medium durch den zweiten Medienanschluss 36 in den distalen Abschnitt 20 der Kammer 12. Befindet sich der Kolben 24 nicht an seinem distalen Totpunkt, so befindet sich das Ventil 44 in einem Durchlasszustand. Die Mittel 34 zur Führung von Flüssigkeiten und/oder Fettgewebe umfassen in diesem Ausführungsbeispiel eine Vorkammer 46. Die Vorkammer 46 weist eine manuell verschließbare Bypassöffnung 48 auf. Ferner weist die Vorkammer 46 eine Kanüle 50 auf. Je nach Anwendungszweck sind unterschiedliche Kanülen an der Vorkammer 46 oder auch direkt an dem proximalen Abschnitt 18 montierbar. In diesem Ausführungsbeispiel umfassen die Mittel 34 mit der Vorkammer 46 zudem einen Vorfilter 52 zur Ausfilterung von Bindegewebsrückständen. Dieser weist eine Durchlassgrenze von 1,5 mm auf.

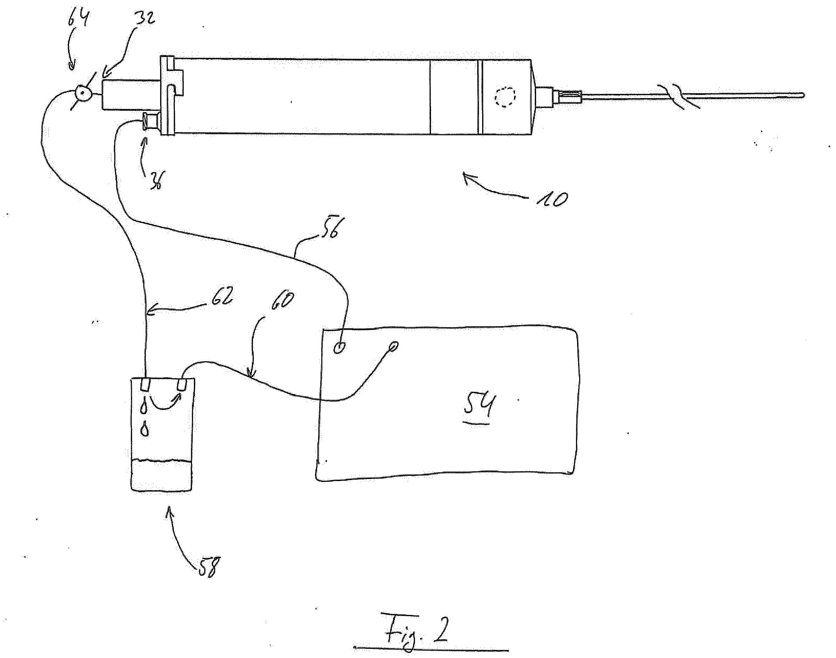

Figur 2 zeigt eine schematische Darstellung eines erfindungsgemäßen Systems, umfassend wenigstens eine erfindungsgemäße Vorrichtung zur Transplantation von Körperfett 10 und wenigstens eine Medienversorgungseinrichtung 54. Sofern Bezugszeichen verwendet werden, die mit denen der Figur 1 identisch sind, so beschreiben diese gleiche Merkmale und gelten zumindest ergänzend auch für Figur 2. Figur 2 zeigt die Vorrichtung 10 und die Medienversorgungseinrichtung 54. Die Medienversorgungseinrichtung 54 ist über eine erste Leitung 56 mit dem zweiten Medienanschluss 36 der Vorrichtung 10 verbunden. Die Medienversorgungseinrichtung 54 ist ausgebildet, die Vorrichtung 10 über die Leitung 56 mit Umgebungsdruck oder einem beliebigen Überdruck, insbesondere aber zwischen 0,5 bar und 30 bar, zu versorgen. Das System umfasst weiterhin Mittel 58 zur Trennung von gasförmigen Medienströmen und flüssigen Stoffströmen. Die Mittel 58 sind zwischen die Vorrichtung 10 und die Medienversorgungseinrichtung 54 geschaltet und mit diesen wirkverbunden. Über die Mittel 58 ist die Medienversorgungseinrichtung 54 über eine zweite Leitung 60 und eine dritte Leitung 62 mit der Vorrichtung 10 verbunden. Die Leitung 62 ist dabei mit dem ersten schließbaren Medienanschluss 32 der Vorrichtung 10 verbunden. Ein schaltbares Absperrventil 64 ist hier prinziphaft dargestellt. Die Medienversorgungseinrichtung 54 ist ausgebildet, die Vorrichtung 10 über die Leitungen 60, 62 mit Umgebungsdruck oder einem beliebigen Unterdruck, insbesondere aber zwischen -900 mbar und -500 mbar, zu versorgen.

### Bezugszeichenliste

- 10: Vorrichtung zur Transplantation von Körperfett
- 12: hohlzylinderförmige Kammer
- 14: Stempel
- 16: innere Mantelfläche
- 18: proximaler Abschnitt
- 20: distaler Abschnitt
- 22: Dichtlippen
- 24: Kolben
- 26: distales Ende
- 28: strömungstechnische Verbindung
- 30: Filter
- 32: erster schließbarer Medienanschluss
- 34: Mittel zur Führung von Flüssigkeiten und/oder Fettgewebe
- 36: zweiter Medienanschluss
- 38: weitere strömungstechnische Verbindung
- 40: äußere Mantelfläche
- 42: Austrittsöffnung
- 44: Ventil
- 46: Vorkammer
- 48: Bypassöffnung
- 50: Kanüle
- 52: Vorfilter
- 54: Medienversorgungseinrichtung
- 56: Leitung
- 58: Mittel zur Trennung von gasförmigen Medienströmen und flüssigen Stoffströmen
- 60: Leitung
- 62: Leitung
- 64: schaltbares Absperrventil

## Patentansprüche

1. Vorrichtung (10) zur Transplantation von Körperfett, umfassend eine zumindest abschnittsweise hohlzylinderförmige Kammer (12), die durch einen an einer inneren Mantelfläche (16) der Kammer (12) führbaren Stempel (14) in einen proximalen Abschnitt (18) und einen von diesem strömungstechnisch entkoppelten distalen Abschnitt (20) unterteilt wird, wobei sich an den Stempel (14) ein Kolben (24) anschließt, der ein distales Ende (26) umfasst, welches eine durch den Stempel (14) verlaufende strömungstechnische Verbindung (28) zu dem proximalen Abschnitt (18) der Kammer (12) aufweist, und der Stempel (14) im Bereich der strömungstechnischen Verbindung (28) zumindest abschnittsweise als Filter (30) ausgebildet ist und das distale Ende (26) des Kolbens (24) einen ersten schließbaren Medienanschluss (32) umfasst und weiterhin an dem proximalen Abschnitt (18) der Kammer (12) Mittel (34) zur Führung von Flüssigkeiten und/oder Fettgewebe angeschlossen oder anschließbar sind, wobei durch Versorgung des ersten Medienanschlusses (32) in geöffnetem Zustand mit einem Unterdruck Flüssigkeit und/oder Fettgewebe in den proximalen Abschnitt (18) der Kammer (12) einsaugbar sind und/oder Flüssigkeit durch den Stempel (14) hindurch einsaugbar ist,
**dadurch gekennzeichnet, dass**
der distale Abschnitt (20) der Kammer (12) einen zweiten Medienanschluss (36) umfasst, so dass durch Versorgung des zweiten Medienanschlusses (36) mit einem Überdruck, während der erste Medienanschluss (32) geschlossen ist, Flüssigkeit und/oder Fettgewebe über eine Verlagerung des Stempels (14) durch den proximalen Abschnitt (18) der Kammer (12) auspumpbar sind,
und/oder dass durch Versorgung des zweiten Medienanschlusses (36) mit Überdruck, während der erste Medienanschluss (32) zumindest geöffnet ist, Flüssigkeit und/oder Fettgewebe über die Verlagerung des Stempels (14) durch den proximalen Abschnitt (18) der Kammer (12) auspumpbar sind und simultan Flüssigkeit durch den Stempel (14) hindurch abführbar ist.

2. Vorrichtung nach Anspruch 1, **dadurch gekennzeichnet, dass** der Stempel (14) wenigstens eine weitere strömungstechnische Verbindung (38) zwischen einer äußeren Mantelfläche (40) des Stempels (14) und einer Austrittsöffnung (42) am distalen Ende (26) des Kolbens (24) aufweist.

3. Vorrichtung nach Anspruch 1 oder 2, **dadurch gekennzeichnet, dass** als Mittel (34) zur Führung von Flüssigkeiten und/oder Fettgewebe wenigstens eine Vorkammer (46) mit wenigstens einer Bypassöffnung (48) und/oder wenigstens eine Kanüle (50) oder Kanüle mit Bypassöffnung an den proximalen Abschnitt (18) der Kammer (12) angeschlossen oder anschließbar sind.

4. Vorrichtung nach Anspruch 3, **dadurch gekennzeichnet, dass** die Vorkammer (46) wenigstens einen Vorfilter (52) zur Ausfilterung von Bindegewebsrückständen umfasst.

5. Vorrichtung nach einem der Ansprüche 1 bis 4, **dadurch gekennzeichnet, dass** der zweite Medienanschluss (36) ein Ventil (44) umfasst, welches sich in einem Sperrzustand befindet, wenn der Kolben (24) sich an einem distalen Totpunkt befindet, und sich in einem Durchlasszustand befindet, wenn der Kolben (24) sich nicht an einem distalen Totpunkt befindet.

6. Vorrichtung nach einem der Ansprüche 1 bis 5, **dadurch gekennzeichnet, dass** der Kolben (24) an einem distalen Totpunkt durch Drehung um seine Längsachse verrastbar ist.

7. Vorrichtung nach einem der Ansprüche 1 bis 6, **dadurch gekennzeichnet, dass** eine Durchlassgrenze wenigstens eines als Filter (30) ausgebildeten Abschnitts des Stempels (14) zwischen 100 µm und 300 µm liegt und/oder eine Durchlassgrenze wenigstens eines Vorfilters (52) zwischen 0,5 mm und 3 mm liegt.

8. Vorrichtung nach einem der Ansprüche 1 bis 7, **dadurch gekennzeichnet, dass** der zweite Medienanschluss (36) mit einem Überdruck zwischen 0,5 bar und 30 bar beaufschlagbar ist und/oder der erste Medienanschluss (32) mit einem Unterdruck zwischen - 900 mbar und -300 mbar beaufschlagbar ist.

9. System, umfassend wenigstens eine Vorrichtung (10) zur Transplantation von Körperfett nach einem der Ansprüche 1 bis 8 und wenigstens eine Medienversorgungseinrichtung (54).

10. System nach Anspruch 9, weiterhin umfassend Mittel (58) zur Trennung von gasförmigen Medienströmen und flüssigen Stoffströmen, die in einer Wirkverbindung zwischen der Vorrichtung (10)zur Transplantation von Körperfett und der Medienversorgungseinrichtung (54) angeordnet sind.

## Claims

1. A device (10) for transplantation of adipose tissue, comprising a chamber (12) which, at least in sections, is in the form of a hollow cylinder, said chamber being divided by a plunger (14) which can be guided on an inner casing surface (16) of the chamber (12) into a proximal portion (18) and a distal portion (20) which is fluidically decoupled from said proximal portion, wherein a piston (24) is connected to the plunger (14), said piston comprising a distal end (26) which has a fluidic connection (28) extending through the plunger (14) to the proximal portion (18) of the chamber (12), and the plunger (14) is configured, at least in sections, as a filter (30) in the region of the fluidic connection (28), and the distal end (26) of the piston (24) comprises a first closable media connection (32) and, furthermore, means (34) for guiding fluids and/or fatty tissue are or can be connected to the proximal portion (18) of the chamber (12), wherein fluid and/or fatty tissue can be sucked into the proximal portion (18) of the chamber (12) and/or fluid can be sucked in through the plunger (14) by supplying the first media connection (32) in the open state with a negative pressure,
**characterized in that**
the distal portion (20) of the chamber (12) comprises a second media connection (36), so that fluid and/or fatty tissue can be pumped out by means of a displacement of the plunger (14) through the proximal portion (18) of the chamber (12) by supplying the second media connection (36) with a positive pressure while the first media connection (32) is closed,
and/or that fluid and/or fatty tissue can be pumped out by means of the displacement of the plunger (14) through the proximal portion (18) of the chamber (12) and, simultaneously, fluid can be discharged through the plunger (14) by supplying the second media connection (36) with a positive pressure while the first media connection (32) is at least open.

2. The device according to Claim 1, **characterized in that** the plunger (14) has at least one additional fluidic connection (38) between an outer casing surface (40) of the plunger (14) and an outlet opening (42) at the distal end (26) of the piston (24).

3. The device according to Claim 1 or 2, **characterized in that** at least one antechamber (46) having at least one bypass opening (48) and/or at least one cannula (50) or cannula having a bypass opening is/are or can be connected to the proximal portion (18) of the chamber (12) as means (34) for guiding fluids and/or fatty tissue.

4. The device according to Claim 3, **characterized in that** the antechamber (46) comprises at least one prefilter (52) for filtering out connective tissue residues.

5. The device according to any one of Claims 1 to 4, **characterized in that** the second media connection (36) comprises a valve (44) which is in a flow-blocking state when the piston (24) is located in a distal dead-center position, and is in a flow-enabling state when the piston (24) is not located in a distal dead-center position.

6. The device according to any one of Claims 1 to 5, **characterized in that** the piston (24) can be latched in a distal dead-center position by being rotated about its longitudinal axis.

7. The device according to any one of Claims 1 to 6, **characterized in that** a transmission limit of at least one portion of the plunger (14) which is configured as a filter (30) is between 100 µm and 300 µm and/or a transmission limit of at least one prefilter (52) is between 0.5 mm and 3 mm.

8. The device according to any one of Claims 1 to 7, **characterized in that** the second media connection (36) can be acted upon with a positive pressure between 0.5 and 30 bar and/or the first media connection (32) can be acted upon with a negative pressure between -900 mbar and -300 mbar.

9. A system, comprising at least one device (10) for transplantation of adipose tissue according to any one of Claims 1 to 8 and at least one media supply device (54).

10. The system according to Claim 9, further comprising means (58) for separating gaseous media flows and liquid material flows, which are arranged in an operative connection between the device (10) for transplantation of adipose tissue and the media supply device (54).

## Revendications

1. Dispositif (10) pour la transplantation de tissu adipeux, comprenant une chambre (12) en forme de cylindre creux au moins par sections, laquelle est divisée par un bouchon (14) pouvant être conduit le long d'une surface d'enveloppe intérieure (16) de la chambre (12) dans une section proximale (18) et dans une section distale (20) découplée de celle-ci du point de vue de l'écoulement, un piston (24) étant raccordé au bouchon (14), lequel piston comprend une extrémité distale (26), laquelle présente une liaison d'écoulement (28), passant par le bouchon (14), avec la section proximale (18) de la chambre (12), et le bouchon (14) étant formé au moins par sections comme filtre (30) dans la zone de la liaison d'écoulement (28) et l'extrémité distale (26) du piston (24) comprenant un premier raccordement de fluides (32) pouvant être fermé et en outre, sur la section proximale (18) de la chambre (12), des moyens (34) pour le guidage de liquides et/ou de tissus adipeux étant raccordés et/ou pouvant être raccordés, du liquide et/ou du tissu adipeux pouvant être aspiré dans la section proximale (18) de la chambre (12) et/ou du liquide pouvant être aspiré à travers le bouchon (14), par application d'une sous-pression au premier raccordement de fluides (32) en état ouvert,
**caractérisé en ce que**
la section distale (20) de la chambre (12) comprend un deuxième raccordement de fluides (36), de telle sorte que du liquide et/ou du tissu adipeux peuvent être pompés par un déplacement du bouchon (14) à travers la section proximale (18) de la chambre (12), par application d'une surpression au deuxième raccordement de fluides (36), pendant que le premier raccordement de fluides (32) est fermé,
et/ou **en ce que** du liquide et/ou du tissu adipeux peut être pompé par le déplacement du bouchon (14) dans la section proximale (18) de la chambre (12) pendant que le premier raccordement de fluides (32) est au moins ouvert, par application d'une surpression au deuxième raccordement de fluides (36), et du liquide pouvant simultanément être évacué à travers le bouchon (14).

2. Dispositif selon la revendication 1, **caractérisé en ce que** le bouchon (14) présente au moins une autre liaison d'écoulement (38) entre une surface d'enveloppe externe (40) du bouchon (14) et une ouverture de sortie (42) sur l'extrémité distale (26) du piston (24).

3. Dispositif selon la revendication 1 ou 2, **caractérisé en ce qu'**au moins une antichambre (46) avec au moins une ouverture de pontage (48) et/ou au moins une canule (50) ou une canule avec ouverture de pontage est raccordée ou peut être raccordée sur la section proximale (18) de la chambre (12), comme moyen (34) pour le guidage de liquides et/ou de tissu adipeux.

4. Dispositif selon la revendication 3, **caractérisé en ce que** l'antichambre (46) comprend au moins un préfiltre (52) pour le filtrage de restes de tissu conjonctif.

5. Dispositif selon l'une des revendications 1 à 4, **caractérisé en ce que** le deuxième raccordement de fluides (36) comprend une valve (44), lequel se trouve en état de fermeture lorsque le piston (24) se trouve à un point mort distal, et se trouve en état de passage lorsque le piston (24) ne se trouve pas à un point mort distal.

6. Dispositif selon l'une des revendications 1 à 5, **caractérisé en ce que** le piston (24), à un point mort distal, est enclenchable par rotation sur son axe longitudinal.

7. Dispositif selon l'une des revendications 1 à 6, **caractérisé en ce qu'**une limite de passage d'au moins une section formée comme filtre (30) du bouchon (14) est située entre 100 µm et 300 µm et/ou une limite de passage d'au moins un préfiltre (52) est située entre 0,5 mm et 3 mm.

8. Dispositif selon l'une des revendications 1 à 7, **caractérisé en ce qu'**une surpression entre 0,5 bar et 30 bar peut être appliquée au deuxième raccordement de fluides (36) et/ou **en ce qu'**une sous-pression entre -900 mbar et -300 bar peut être appliquée au premier raccordement de fluides (32).

9. Système comprenant au moins un dispositif (10) pour la transplantation de tissu adipeux selon l'une des revendications 1 à 8 et au moins une installation d'alimentation en fluides (54).

10. Système selon la revendication 9, comprenant en outre des moyens (58) pour la séparation de flux de fluides gazeux et de flux de matières liquides, lesquels sont disposés en relation active entre le dispositif (10) pour la transplantation de tissu adipeux et l'installation d'alimentation en fluides (54).
